(19) Europäisches Patentamt
European Patent Office
Office européen des brevets

(11) Publication number : 0 460 924 A1

(12) **EUROPEAN PATENT APPLICATION**

(21) Application number : 91305057.1

(51) Int. Cl.[5] : C07D 213/30, A61K 31/44

(22) Date of filing : 04.06.91

(30) Priority : 05.06.90 GB 9012465
05.03.91 GB 9104645

(43) Date of publication of application :
11.12.91 Bulletin 91/50

(84) Designated Contracting States :
AT CH DE DK FR GB IT LI NL SE

(71) Applicant : GLAXO GROUP LIMITED
Clarges House 6-12 Clarges Street
London W1Y 8DH (GB)

(72) Inventor : Judkins, Brian David
Glaxo Group Research Limited, Park Road, Ware
Hertfordshire, SG12 0DG (GB)
Inventor : Evans, Brian
Glaxo Group Research Limited, Park Road, Ware
Hertfordshire, SG12 0DG (GB)
Inventor : Meadows, James David
Glaxo Group Research Limited, Berkley Avenue
Greenford, Middlesex UB6 0HE (GB)

(74) Representative : Marchant, James Ian et al
Elkington & Fife, Prospect House, 8 Pembroke Road
Sevenoaks, Kent TN13 1XR (GB)

(54) **Dichloroaniline compound.**

(57) The present invention provides the compound (R)-(-)-4-amino-3,5-dichloro-α-[[[6-[2-(2-pyridinyl)ethoxy]hexyl]amino] methyl]benzenemethanol or physiologically acceptable salts thereof, processes for its preparation, pharmaceutical formulations containing it, and its use in medicine.

EP 0 460 924 A1

Jouve, 18, rue Saint-Denis, 75001 PARIS

This invention relates to an optically active dichloroaniline derivative having a stimulant action at $ß_2$-adrenoreceptors, to processes for its preparation, to pharmaceutical compositions containing it and to its use in medicine.

In our British Patent Specification No 2187734A we have described a novel class of phenethanolamine derivatives, including the racemic mixture of the compound 4-amino-3,5-dichloro-α-[[[6-[2-(2-pyridinyl)ethoxy]hexyl]amino]methyl]benzenemethanol (hereinafter referred to as "Compound A"), having stimulant activity at $ß_2$-adrenoreceptors.

We now provide an optical isomer of Compound A.

Thus, according to one aspect of the present invention we provide the compound: (R)-(-)-4-amino-3,5-dichloro-α-[[[6-[2-(2-pyridinyl)ethoxy] hexyl]amino]methyl]benzenemethanol, and its physiologically acceptable salts.

The (R)-isomer of Compound A is provided substantially free of the corresponding (S)-isomer.

As used herein, the term "substantially free" means that the (R)-isomer has an enantiomeric excess of at least 90%, preferably 96%, and in particular 98%.

As used herein, "enantiomeric excess" is an indication of the relative proportion of one enantiomer to the other, and is simply the percentage difference between the ratio of the two enantiomers. Thus, for example, a 75:25 ratio of enantiomers is equivalent to a 50% enantiomeric excess and a 95:5 ratio is equivalent to a 90% enantiomeric excess.

The compound according to the invention has a selective stimulant action at $ß_2$-adrenoreceptors, which furthermore is of a particularly advantageous profile. The stimulant action may be demonstrated in the isolated trachea of the guinea-pig where the compound causes relaxation of contractions induced by $PGF_2α$ or electrical stimulation. The compound also has a prolonged duration of action.

The compound according to the invention may be used in the therapy or prophylaxis of conditions susceptible to amelioration by a compound possessing selective stimulant action at $ß_2$-adrenoreceptors, particularly of diseases associated with reversible airways obstruction such as asthma and chronic bronchitis. The compound according to the invention may also be used in the therapy or prophylaxis of inflammation, allergy or allergic reaction. Further examples of conditions which may be alleviated by administration of a compound possessing selective $ß_2$-stimulant activity are inflammatory and allergic skin diseases, depression, premature labour, glaucoma and conditions in which there is an advantage in lowering gastric acidity, particularly in gastric and peptic ulceration.

In a further or alternative aspect of the invention, therefore, we provide the (R)-isomer of Compound A or a physiologically acceptable salt thereof for use in medicine, more particularly for use in the treatment of conditions which may be ameliorated by administration of a compound having a selective stimulant action at $ß_2$-adrenoreceptors.

In another aspect, the invention further provides a method for the treatment of a condition subject to amelioration by a compound having selective stimulant activity at $ß_2$-adrenoreceptors in a mammal, including a human, comprising administration of an effective amount of the (R)-isomer of Compound A or a physiologically acceptable salt thereof.

There is also provided in a further or alternative aspect use of the (R)-isomer of Compound A or a physiologically acceptable salt thereof, for the manufacture of a medicament for the treatment of a condition which may be ameliorated by a compound having selective $ß_2$-adrenoreceptor stimulant activity.

It is to be understood that references herein to treatment may extend to prophylaxis as well as the treatment of established conditions.

Suitable physiologically acceptable salts of the compound of the invention include acid addition salts derived from inorganic and organic acids, such as hydrochlorides, hydrobromides, sulphates, phosphates, maleates, tartrates, citrates, benzoates, 4-methoxybenzoates, 2- or 4-hydroxybenzoates, 4-chlorobenzoates, benzenesulphonates, p-toluenesulphonates, naphthalenesulphonates, methanesulphonates, sulphamates, ascorbates, salicylates, acetates, diphenylacetates, triphenylacetates, adipates, fumarates, succinates, lactates, glutarate, gluconates, tricarballylates, hydroxynaphthalenecarboxylates e.g 1-hydroxy or 3-hydroxy-2-naphtalenecarboxylates, or oleates.

It is possible that the compound of the invention may be administered to a patient as the raw chemical, but it is preferable to present the active ingredient as a pharmaceutical formulation.

The invention accordingly provides a pharmaceutical formulation comprising the (R)-isomer of Compound A or a physiologically acceptable salt thereof together with one or more physiologically acceptable carriers and, optionally, other therapeutic and/or prophylactic ingredients. The carrier(s) must be "acceptable" in the sense of being compatible with the other ingredients of the formulation and not deleterious to the recipient thereof.

The compound may be formulated in a form suitable for administration by inhalation or insufflation, or for oral, buccal, parenteral, topical (including nasal) or rectal administration. Administration by inhalation or insufflation is preferred.

For administration by inhalation the compound according to the invention are conveniently delivered in the form of an aerosol spray presentation from pressurised packs, with the use of a suitable propellant, such as a chlorofluorocarbon (e.g. dichlorodifluoromethane, trichlorofluoromethane, dichlorotetrafluoroethane), a hydrogen-containing fluorocarbon or chlorofluorocarbon (e.g. chlorofluoromethane, 1,1,1,2-tetrafluoromethane or 1,1,1,2,3,3,3-heptafluoro-n-propane) or mixtures thereof, carbon dioxide or other suitable gas, or from a nebuliser. In the case of a pressurised aerosol the dosage unit may be determined by providing a valve to deliver a metered amount.

Alternatively, for administration by inhalation or insufflation, the compound according to the invention may take the form of a dry powder composition, for example a powder mix of the compound and a suitable powder base such as lactose or starch. The powder composition may be presented in unit dosage form in for example capsules or cartridges of e.g. gelatin, or blister packs from which the powder may be administered with the aid of an inhaler or insufflator.

For oral administration, the pharmaceutical composition may take the form of, for example, tablets, capsules, powders, solutions, syrups or suspensions prepared by conventional means with acceptable excipients.

For buccal administration the composition may take the form of tablets, drops or lozenges formulated in the conventional manner.

The compound of the invention may be formulated for parenteral administration by bolus injection or continuous infusion. Formulation for injection may be presented in unit dosage form in ampoules, or in multi-dose containers with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle, e.g. sterile pyrogen-free water, before use.

For topical administration the pharmaceutical composition may take the form of ointments, lotions or creams formulated in a conventional manner, with for example an aqueous or oily base, generally with the addition of suitable thickening agents and/or solvents. For nasal application, the composition may take the form of a spray, formulated for example as an aqueous solution or suspension or as an aerosol with the use of a suitable propellant.

The compound of the invention may also be formulated in rectal compositions such as suppositories or retention enemas, e.g. containing conventional suppository bases such as cocoa butter or other glyceride.

Where pharmaceutical compositions are described above for oral, buccal, rectal or topical administration, these may be presented in a conventional manner associated with controlled release forms.

A proposed daily dosage of active compound for the treatment of man is 0.005mg to 100mg, which may be conveniently administered in one or two doses. The precise dose employed will of course depend on the age and condition of the patient and on the route of administration. Thus a suitable dose for administration by inhalation is 0.005mg to 20mg, for oral administration is 0.02mg to 100mg, and for parenteral administration is 0.01mg to 2mg for administration by bolus injection and 0.01mg to 25mg for administration by infusion.

The compound of the invention may be prepared according to the general methods described in British Patent Specification No. 2187734A from starting materials having the desired stereochemical configuration. In particular, the compound of the invention may be prepared by a number of processes as described below. In the general processes (B) to (D) described below, the final step may be the removal of a protecting group.

Thus, according to a further aspect of the present invention we provide a process (A) for preparing the (R)-isomer of Compound A by deprotection of a compound of formula (I) :

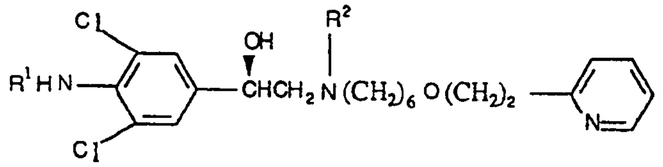

(I)

wherein $R^1$ and $R^2$ each independently represents a hydrogen atom or a protecting group with the proviso that at least one of them represents a protecting group.

The protecting groups may be any conventional protecting groups, for example as described in "Protective Groups in Organic Synthesis" by Theodora Greene (John Wiley and Sons Inc. 1981). Examples of suitable amino protecting groups represented by $R^1$ and $R^2$ include aralkyl groups such as benzyl, α-methylbenzyl, α-hydroxymethylbenzyl, diphenylmethyl or triphenylmethyl groups and acyl groups such as acetyl, trichloroacetyl or trifluoroacetyl.

In a preferred embodiment of process (A), $R^2$ is a protecting group which contains an asymmetric centre, for example $R^2$ may represent the group

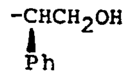 or 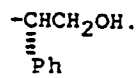

The deprotection to yield the (R)-isomer of Compound A may be effected using conventional techniques. Thus, for example, when $R^1$, and/or $R^2$ is a substituted or unsubstituted aralkyl group this may be cleaved by hydrogenolysis in the presence of a metal catalyst (e.g. palladium on charcoal). Acyl groups may be removed by hydrolysis with an acid such as mineral acid, e.g. hydrochloric acid, or a base such as sodium hydroxide or potassium carbonate.

Intermediates of formula (I) wherein $R^2$ is the group

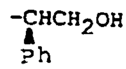

may be prepared by reduction of compounds of formula (IIR)

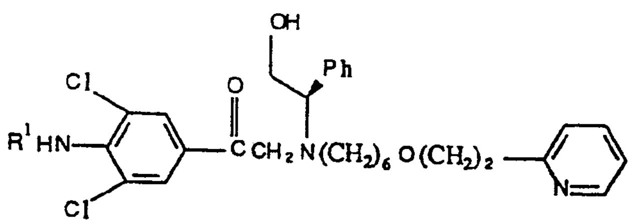

(IIR)

wherein $R^1$ is as defined for formula (I), using a hydride such as diborane or a complex metal hydride such as lithium aluminium hydride, lithum borohydride or preferably sodium borohydride in a suitable solvent, such as an alcohol, for example, methanol, or an ether, for example, 1,2-dimethoxyethane, or a mixture thereof, preferably at elevated temperature.

Similarly, intermediates of formula (I) wherein $R^2$ is the group

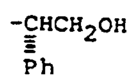

may be analogously prepared from compounds of

formula (IIS)

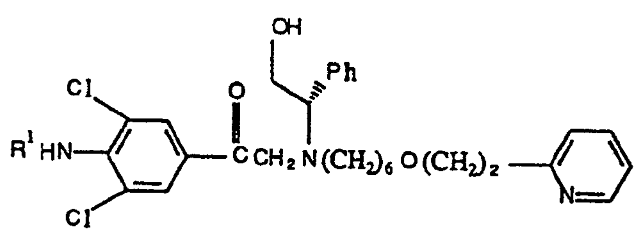

(IIS)

wherein $R^1$ is defined as above.

It will be appreciated that reduction of a compound of formula (IIR) or (IIS) will yield a mixture of two diastereoisomers the separation of which may therefore be effected at any time prior to the removal of the (R)- or (S)-2-hydroxy-1-phenylethyl group.

Compounds of formula (IIR) may be prepared by reaction of a compound of formula (III)

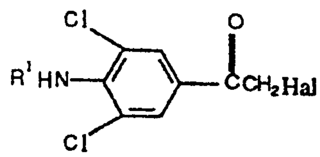

(III)

wherein X and $R^1$ are as defined above and Hal represents a halogen atom, for example a chlorine or iodine atom or preferably a bromine atom, with the compound of formula (IVR)

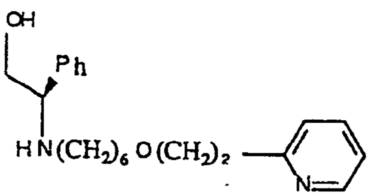

(IVR)

The reaction is effected in a suitable solvent, such as acetonitrile or an ether, for example, tetrahydrofuran or 1,2-dimethoxyethane, at a temperature between ambient and the reflux temperature of the solvent, and preferably in the presence of a base such as diisopropylethylamine, sodium carbonate or other acid scavenger such as propylene oxide.

Compounds of formula (IIS) may be analogously prepared from a compound of formula (III) and the compound of formula (IVS)

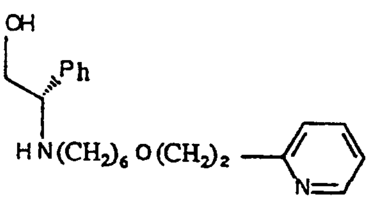

(IVS)

Intermediates of formula (IVR) may be prepared from a compound of formula (V)

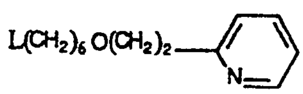

(V)

wherein L represents a leaving group, for example a halogen atom such as chlorine, bromine or iodine, or a hydrocarbylsulphonyloxy group such as methanesulphonyloxy or p-toluenesulphonyloxy, by reaction with (R)-phenylglycinol. The reaction is preferably effected in the presence of a suitable acid scavenger, for example a base such as diisopropyethylamine conveniently in a solvent such as acetonitrile or an ether, e.g. tetrahydrofuran, a substituted amide, e.g. dimethylformamide or a chlorinated hydrocarbon, e.g. chloroform at a temperature between ambient and the reflux temperature of the solvent.

Intermediates of formula (IVS) may be prepared analogously from a compound of formula (V) and (S)-phenylglycinol.

The preparation of compounds of formulae (III) and (V) is described in British Patent Specification No. 2187734A.

Alternatively compounds of formula (I) wherein $R^2$ is the group

$$-\underset{Ph}{CH}CH_2OH$$

may be prepared by reduction of a compound of formula (VI)

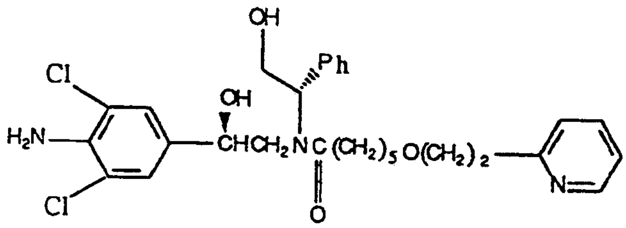

(VI)

using the conditions described, for example, in process (C) below, followed, where necessary, by the removal of the 2-hydroxy-1-phenylethyl group.

The compound of formula (VI) may be prepared by acylation of a compound of formula (VII)

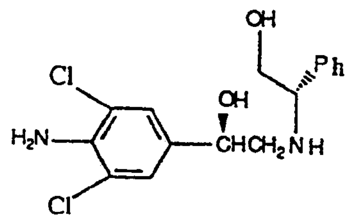

(VII)

using an activated carboxylic acid of formula (VIII)

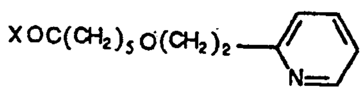

(VIII)

(wherein X represents a leaving group e.g. chlorine). The acid chloride may be prepared by reaction of the acid corresponding to formula (VIII) with phosphorous pentachloride, thionyl chloride or oxalyl chloride, in a solvent such as a substituted amide, e.g. dimethylformamide, preferably at room temperature.

The acylation reaction may be carried out in the presence of an organic base, for example, triethylamine or pyridine in a non-aqueous medium, such as acetonitrile, dimethylformamide or tetrahydrofuran at a temperature within the range -25°C to +150°C.

The compound of formula (VII) may be obtained by reaction of a compound of formula (III) (wherein $R^1$ is a hydrogen atom) with (S)-phenylglycinol. The reaction is preferably effected in the presence of a suitable acid scavenger such as N,N-diisopropylethylamine, conveniently in a solvent such as acetonitrile or an ether e.g. tetrahydrofuran at a temperature between room temperature and the reflux temperature of the solvent. Subsequent treatment of the reaction mixture in an alcoholic (e.g. methanolic) solvent with a hydride such as sodium borohydride in the presence of a catalyst (e.g. calcium chloride) yields the compound of formula (VII).

Reaction of a compound of formula (III) with (R)-phenylglycinol, followed by reduction with sodium borohydride in the presence of calcium chloride gives predominantly the reverse isomer of the compound of formula (VII).

Intermediates of formula (I) for use in the deprotection process may alternatively be prepared by reaction of a chiral epoxide of formula (IX)

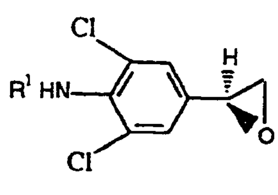

(IX)

(wherein $R^1$ is as defined above) with an amine of formula (X)

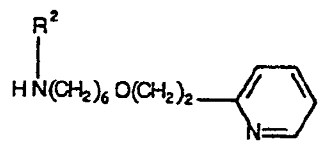

(X)

(wherein $R^2$ is as defined in formula (I)) conveniently in a suitable solvent, such as an alcohol, for example methanol, at a temperature between ambient and the reflux temperature of the solvent Epoxides of formula (IX) may be prepared from appropriate chiral starting materials by procedures well known in the art, for example, by procedures analogous to those described in British Patent Specification No. 2140800A.

In another general process (B) the (R)-isomer of Compound A may be obtained by the reaction of a compound of general formula (XI)

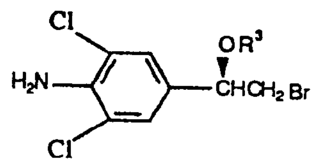

(XI)

(wherein $R^3$ is a suitable hydroxyl protecting group such as an ether, for example, a silyl ether (for example, a triethylsilyl or t-butyldimethylsilyl group), with a compound of formula (XII)

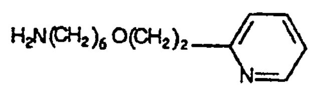

(XII)

The reaction is preferably effected in the presence of a suitable acid scavenger, for example, a weak inorganic base such as sodium bicarbonate or an organic base such as diisopropylethylamine or triethylamine. The reaction is also preferably effected in the presence of sodium iodide, conveniently in a solvent such as acetonitrile or an ether e.g. tetrahydrofuran, a ketone e.g. butanone, a substituted amide e.g. dimethylformamide, an aromatic hydrocarbon e.g. toluene or a chlorinated hydrocarbon e.g. chloroform, at a temperature between room temperature and the reflux temperature of the solvent.

The reaction may be followed by deprotection of the hydroxyl group by for example, mild acidic hydrolysis (e.g. aqueous acetic acid in THF), alkaline hydrolysis (e.g. potassium carbonate in anhydrous methanol) or using a fluoride (e.g. tetrabutylammonium fluoride or potassium fluoride in an appropriate solvent e.g. THF).

Intermediates of formula (XI) may be prepared by a chiral reduction of the bromoketone of formula (XIII)

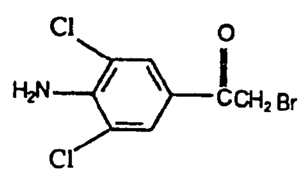

(XIII)

using a boron reducing agent such as borane in the presence of an (R)-1,3,2-oxazaborolidine chiral catalyst, such as (R)-5,5-diphenyl-2-methyl-3,4-propano-1,3,2-oxazaborolidine or (R)-5,5-diphenyl-4-isopropyl-2-methyl-1,3,2-oxazaborolidine (see, for instance, E.J. Corey et al., J Amer. Chem. Soc., (1987), 109, pp5551-5553) followed by protection with, for example, a silyl ether. The reduction is conveniently effected in a solvent such as an ether, for example, tetrahydrofuran, at a temperature in the range between -20°C and 50°C, for example, between 0°C and room temperature.

Formation of the silyl ether of formula (XI) is effected using an appropriate silyl halide (e.g. chloride) such as t-butyldimethylsilyl chloride or chlorotriethylsilane, in the presence of a base such as an organic base (e.g. imidazole) in a suitable solvent such as a substituted amide (e.g. dimethylformamide) or an ether (e.g. tetrahydrofuran).

The preparation of intermediates of formulae (XII) and (XIII) is described in British Patent Specification No. 2187734A.

In another general process (C) the (R)-isomer of Compound A may be obtained by the reduction of a compound of formula (XIV)

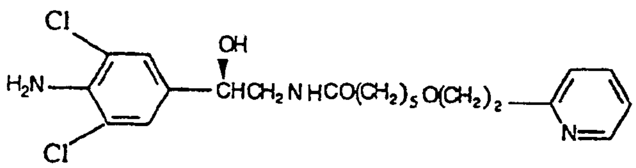

(XIV)

or a protected derivative thereof.

The reduction may be effected using reducing agents conveniently employed for the reduction of amides for example, a hydride such as diborane or a complex metal hydride such as lithium aluminium hydride or sodium bis(2-methoxyethoxy)aluminium hydride, in a solvent such as an ether e.g. tetrahydrofuran or diethyl ether, and at a temperature between room temperature and the reflux temperature of the solvent.

Intermediates of formula (XIV) may be obtained by conventional amide formation between a compound of formula (XV)

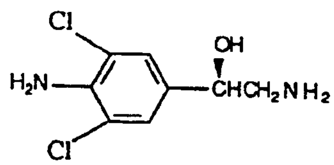

(XV)

and a compound of formula (XVI)

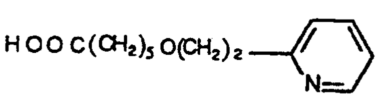

(XVI)

The reaction is preferably effected in the presence of a coupling agent such as N,N[1]-carbonyldiimidazole (CDI) or dicyclohexanecarbodiimide, in a solvent such as an ether, for example, tetrahydrofuran at a temperature between 0°C and 100°C, for example, room temperature.

The intermediate of formula (XV) may be obtained by chiral reduction of an azide of formula (XVII)

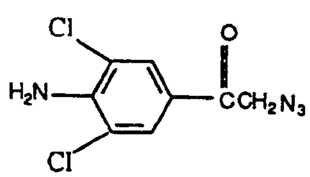

(XVII)

followed by reduction of the azide moiety to an amino group.

The chiral reduction may be effected substantially as described for the preparation of a compound of formula (XIII), above. Reduction of the azide may be effected using hydrogen in the presence of a catalyst such as palladium or palladium oxide on a support such as charcoal using an alcohol, e.g. ethanol as a solvent.

The azide of formula (XVII) may be prepared in a conventional manner by treatment of the compound of formula (XIII) or its racemate with sodium azide in a solvent such as an alcohol, e.g. ethanol, an ether, e.g. tetrahydrofuran, water or a mixture of solvents.

The preparation of the compound of formula (XVI) is described in British Patent Specification No. 2187734A.

In another general process (D) the (R)-isomer of Compound A may be obtained by alkylation of the chiral amine of formula (XVIII)

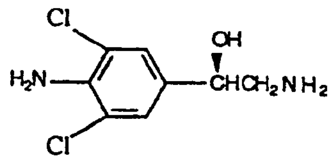

(XVIII)

using an alkylating agent of formula (XIX)

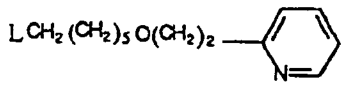

(XIX)

(wherein L is a leaving group, for example a halogen atom such as chlorine, bromine or iodine, or a hydrocarbylsulphonyloxy group such as methanesulphonyloxy or p-toluenesulphonyloxy).

The alkylation is preferably effected in the presence of a suitable acid scavenger, for example, inorganic bases such as sodium or potassium carbonate, organic bases such as triethylamine or N,N-diisopropylethylamine, or alkylene oxides such as ethylene oxide or propylene oxide. The reaction is conveniently effected in a solvent such as acetonitrile or an ether e.g tetrahydrofuran, a ketone e.g. butanone, a substituted amide e.g. dimethylformamide or a chlorinated hydrocarbon e.g. chloroform at a temperature between room temperature and the reflux temperature of the solvent.

In an alternative alkylation reaction, the compound of formula (XVIII) may be reacted with a compound of formula (XX)

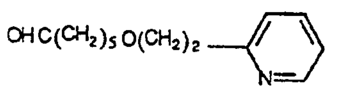

(XX)

in the presence of a reducing agent.

Suitable reducing agents include a hydride such as diborane or a metal hydride such as sodium borohydride or lithium aluminium hydride or hydrogen in the presence of a catalyst such as platinum, platinum oxide, palladium, palladium oxide, Raney nickel or rhodium, on a support such as charcoal, using an alcohol e.g. ethanol or methanol, or an ester e.g. ethyl acetate, or an ether e.g. tetrahydrofuran, or water, as reaction solvent, or a mixture of solvents e.g. a mixture of two or more of those just described, at normal or elevated temperature and pressure, for example, from 20° to 100°C and from 1 to 10 atmospheres.

The compound of formula (XVIII) may be obtained by chiral reduction as previously described herein, or alternatively by chiral resolution of a mixture of the compound of formula (XVIII) and its corresponding (S)-enantiomer by reaction with an optically active acid including carboxylic and sulphonic organic acids, for example(+)- or (-)-tartaric acid or (+)- or (-)-dibenzoyltartaric acid, especially di-p-toluoyl-D-tartaric acid, in a suitable solvent as an alcohol e.g. ethanol, conveniently at an elevated temperature.

Once the enantiomers have been resolved, the desired enantiomer can be recovered as the free base by conventional methods, for example, by reaction with a base, for example, sodium carbonate.

The (R)-isomer of Compound A may also be obtained by resolution of racemic Compound A using conventional methods.

The term "resolution" is used herein in the conventional practical sense used in the art to include partial resolution, that is, the separation of a mixture of enantiomers of a compound (in any ratio) into two fractions, one of which is enriched in one enantiomer relative to the initial mixture.

Resolution of the mixture of (R)- and (S)-isomers of Compound A may be effected conventionally by derivatising the mixture with a chiral derivatising agent, to form a mixture of diastereomers of a derivative of Compound A. The components of the mixture may then be separated conventionally, for example by fractional crystallisation Separation may be complete or partial. The derivatisation is preferably effected by reacting a mixture of the (R)- and (S)-isomers of Compound A with a suitable optically active acid as described above.

The preferred process for the preparation of the (R)-isomer of Compound A is from the chiral protected alcohol of formula (XI) according to general process (B).

The (R)-isomer of Compound A, however prepared, may be converted to its acid addition salts by treatment with a suitable acid. Thus, for example, the (R)-isomer of Compound A may be converted to its fumarate salt by treatment with fumaric acid.

The following examples illustrate the invention.

Temperatures are in °C. Thin layer chromatography (T.l.c.) and flash column chromatography were carried out over silica and "dried" refers to drying using sodium sulphate or magnesium sulphate. THF means tetrahydrofuran.

Example 1

(R)-(-)-4-Amino-3,5-dichloro-a-[[[6-[2-(2-pyridinyl)ethoxy]hexyl]amino] methyl]benzenemethanol, (E)- butenedioate salt (2:1)

(i) (R)-β-[[(6-[2-(2-pyridinyl)ethoxy]hexyl]amino]benzeneethanol

A solution of 2-[2-[(6-bromohexyl)oxy]ethyl]pyridine (1.1g) in dry acetonitrile (10ml) was added to a stirred solution of (R)-phenylglycinol (0.82g), sodium iodide (0.65g) and N,N-diisopropylethylamine (2ml, 1.48g) in dry acetonitrile (70ml) under nitrogen, and refluxed overnight. The solution was evaporated in vacuo, water (50ml) added and the solution extracted with toluene (3x50ml). The combined organic phases were dried, filtered and the filtrate evaporated in vacuo. Purification by flash column chromatography eluting with dichloromethane:methanol:ammonia [200:8:1] gave the title compound as a yellow oil (0.89g).

| | | |
|---|---|---|
| Assay Found: | | C,72.85; H,9.1; N,8.4. |
| $C_{21}H_{30}N_2O_2.0.2H_2O$ requires | | C,72.9; H,8.85; N,8.1%. |

$[\alpha]_D^{20}$ = -37.5° (c 0.7, methanol)

(ii) (R)-1-(4-Amino-3,5-dichlorophenyl)-2-[(2-hydroxy-1-phenylethyl) [6-[2-(2-pyridinyl) ethoxy] hexyl] amino] ethanone

A mixture of 1-(4-amino-3,5-dichlorophenyl)-2-bromoethanone (1.00g), (R)-ß-[[6-[2-(2-pyridinyl) ethoxy] hexyl]amino]benzeneethanol (1.21g), N,N-diisopropylethylamine (2ml) and THF (45ml) was heated at reflux for 6h under nitrogen, cooled and stored at 5° for 65h. The mixture was filtered and evaporated in vacuo and the residue purified by flash chromatography eluting with hexane:ethyl acetate (3:1-1:1) to afford the title compound as a viscous gum (495mg).

T.l.c. (hexane:ethyl acetate 1:1) Rf 0.42

(iii) (-)-α-(R)-4-Amino-3,5-dichloro-α-[[(2-hydroxy-1-phenylethyl) [6- [2-(2-pyridinyl) ethoxy] hexyl] amino] methyl]benzenemethanol

A solution of (R)-1-(4-amino-3,5-dichlorophenyl)-2-[(2-hydroxy-1-phenylethyl)[6-[2-(2-pyridinyl) ethoxy] hexyl]amino]ethanone (0.49g) in methanol (10ml) and 1,2,-dimethoxyethane (50ml) was cooled to 5° and sodium borohydride (0.72g) added portionwise over 0.5h and then allowed to warm to 20° over 17h. The solution was evaporated in vacuo and the residue partitioned between 8% sodium bicarbonate (50ml) and ethyl acetate (100ml) and the organic phase dried and evaporated in vacuo. Another reduction was combined at this stage (from 0.66g of the ethanone intermediate) to give 1.23g residue. Purification by flash chromatography over Sorbsil C-60 (140g) eluting with hexane:isopropanol:triethylamine:dichloromethane (120:5:5:20) afforded the title compound as a colourless gum (86mg) plus mixed fractions (overall yield of both isomers 708mg).
$[\alpha]_D^{20}$ = -16.5° (c 0.7, $CHCl_3$)

| | | |
|---|---|---|
| Analysis | Found: | C,63.8; H,6.9; N,7.8. |
| $C_{29}H_{37}Cl_2N_3O_3$ requires | | C,63.7; H,6.8; N,7.7%. |

H.p.l.c. ratio of enantiomers (R:S) 98.0:2.0 (99% chemically pure).

(iv) (R)-(-)-4-Amino-3,5-dichloro-α-[[[6-2-[2-pyridinyl)ethoxy] hexyl]amino]methyl]benzenemethanol, (E)- butenedioate salt (2:1)

A solution of (-)-α-(R)-4-amino-3,5-dichloro-α-[[(2-hydroxy-1-phenylethyl)[6-[2-(2-pyridinyl)ethoxy]hexyl]amino]methyl] benzenemethanol (co-evaporated with ethanol, 750mg) in ethanol (15ml) was treated with a 1.2M solution of hydrogen chloride in ethanol (2.20ml) and added to a pre-hydrogenated suspension of 10% palladium on charcoal (50% aq. paste, 500mg) in ethanol (15ml) and hydrogenated at room temperature and pressure for 10h. The mixture was filtered through hyflo and evaporated in vacuo and the residue partitioned between 8% sodium bicarbonate (50ml) and ethyl acetate (120ml). The organic phase was dried and evaporated in vacuo and the residue purified by flash chromatography eluting with toluene:ethanol:triethylamine (96:2:2-88:10:2). Trituration of the resulting oil afforded the free base of the title compound (340mg). This was dissolved in warm methanol (20ml), fumaric acid (47mg) added and the solution evaporated in vacuo. The residue was recrystallised from isopropanol (4ml) to afford the title compound as a colourless solid (250mg) m.p. 128.5-130.5°.

Analysis Found: C,56.7; H,6.45; N,8.3; Cl,14.0.

$C_{21}H_{29}Cl_2N_3O_2.0.5C_4H_4O_4.0.2H_2O$ req: C,56.6; H,6.5; N,8.6; Cl,14.50%

H.p.l.c. ratio R:S = 99.4:0.6.

$[\alpha]_D^{20}$ = -25.7° (c 0.8, methanol)

Example 2

(S)-(+)-4-Amino-3,5-dichloro-α-[[[6-[2-(2-pyridinyl)ethoxy]hexyl] amino]methyl]benzenemethanol, (E)-butenedioate salt (2:1)

(i) (+)-α-(R)-4-Amino-3,5-dichloro-α-[[(2-hydroxy-1-phenylethyl)[6- [2-(2-pyridinyl) ethoxy] hexyl] amino] methyl]benzenemethanol

The title compound (1.48g) was prepared in four batches by reduction of the product of Example 1, part (ii) (2.07g in total) according to the method of Example 1, part (iii). The combined batches were purified by flash chromatography over silica (Sorbsil C-60, 150g) eluting with hexane:ethanol:triethylamine:dichloromethane (170:5:5:20) to afford the title compound as a pale yellow viscous gum (1.48g).

Analysis Found: C,63.75; H,7.05; N,7.5.

$C_{29}H_{37}Cl_2N_3O_3$ requires C,63.7; H,6.8; N,7.7%.

$[\alpha]_D^{20}$ = 3.7° (c 0.7, $CHCl_3$).

H.p.l.c. ratio of enantiomers (R:S) = 1.3:98.7.

(ii) (S)-(+)-4-Amino-3,5-dichloro-α-[[[6-[2-(2-pyridinyl)ethoxy] hexyl]amino]methyl]benzenemethanol, (E)-butenedioate salt (2:1)

A solution of (+)-α-(R)-4-amino-3,5-dichloro-α-[[(2-hydroxy-1-phenylethyl)[6-[2-(2-pyridinyl)ethoxy] hexyl] amino]methyl] benzenemethanol (1.338g) (ratio of enantiomers (R:S), 1.3:98.7) in ethanol (25ml) was treated with a 1.15M solution of hydrogen chloride in ethanol (4.2ml) and added to a pre-hydrogenated suspension of 10% palladium on charcoal (50% aqueous paste, 1.2g) in ethanol (15ml) and hydrogenated at room temperature and pressure for 10h. The mixture was filtered through hyflo and evaporated in vacuo to an oil which was partitioned between 8% sodium bicarbonate (20ml) and ethyl acetate (100ml). The organic phase was washed with brine (15ml), dried and evaporated in vacuo and the residue purified by flash chromatography eluting with toluene:ethanol:triethylamine (96:2:2-88:10:2) followed by trituration with hexane (5ml, 2x 10ml) to afford the free base of the title compound (791mg). The material was dissolved in warm methanol (20ml), fumaric acid (179mg) added and the solution evaporated in vacuo. The residue was slowly recrystallised from isopropanol (12ml) to afford the title compound as a colourless powder (425mg) mp 130-131°.

Analysis Found: C,56.7; H,6.5; N,8.4; Cl,14.8.

$C_{21}H_{29}Cl_2N_3O_2.0.5C_4H_4O_4$ requires C,57.0; H,6.45; N,8.7; Cl,14.6%.

H.p.l.c. >99% (S)-enantiomer,

$[\alpha]_D^{20}$ = +27.45$^0$ (c 1.0, methanol)

## Example 3

(R)-(-)-4-Amino-3,5-dichloro-α-[[[6-[2-(2-pyridinyl)ethoxy]hexyl] amino]methyl]benzenemethanol, (E)-butenedioate salt (2:1)

### (i) [R-(R*S*)]-4-Amino-3,5-dichloro-α-[[(2-hydroxy-1-phenylethyl)amino]methyl]benzenemethanol

(S)-Phenylglycinol (2.06g) was dissolved in THF (50ml), and N,N-diisopropylethylamine (3.5ml) and 1-(4-amido-3,5-dichlorophenyl)-2-bromoethanone (2.83g) were added. The solution was left at 21° for 4.5h, then filtered and evaporated in vacuo. The residue in methanol (75ml) was treated with calcium chloride dihydrate (2.9g) and cooled to 0.5°. Sodium borohydride (800mg) was added portionwise over 0.5h at 0-5° with stirring After a further 0.5h the mixture was evaporated in vacuo and the residue partitioned between 2M hydrochloric acid (100ml) and ethyl acetate (2x100ml). The organic extracts were washed with 2M sodium carbonate (100ml), dried and evaporated in vacuo. The residue was purified by flash chromatography eluting with ethyl acetate to give the title compound as a pale pink foam (2.44g).
T.l.c. ethyl acetate:methanol (19:1) Rf 0.31
H.p.l.c. ratio of isomers (R:S) 91.9:8.1

### (ii) 6-[2-(2-Pyridinyl)ethoxy]hexanoyl chloride, hydrochloride

A solution of 6-[2-(2-pyridinyl)ethoxy]hexanoic acid (9.03g) in dichloromethane (120ml) was treated with DMF (1ml) and thionyl chloride (20ml) added slowly (with ice-bath cooling applied). The solution was stirred at room temp for 17h. The solution was evaporated in vacuo then co-evaporated with toluene (x2) to afford the title compound as a dark viscous oil (14g).

### (iii)[R-(R*,S*)]-N-[2-(4-Amino-3,5-dichlorophenyl)-2-hydroxyethyl]-N-(2-hydroxy-1-phenylethyl)-6-[2-(2-pyridinyl) ethoxy]hexanamide

The product of step (i) (0.4g) in acetonitrile (8ml) was treated with triethylamine (0.7ml) and a solution of the product of step (ii) (0.3g) in acetonitrile (3ml) added slowly. The dark mixture was stirred at 21° for 16h then evaporated in vacuo. The residue was taken up in THF (10ml), filtered and evaporated in vacuo. The residue was purified by flash chromatography eluting with hexane:ethyl acetate (1:1). Further elution with ethyl acetate, followed by ethyl acetate:methanol (19:1-9:1) gave the title compound as a viscous gum (380mg).
T.1.c dichloromethane:isopropanol:triethylamine (50:5:1) Rf 0.43.

### (iv) [R-(R*S*)]-4-Amino-3,5-dichloro-α-[[(2-hydroxy-1-phenylethyl)[6- (2-pyridineethoxy) hexyl]amino] methyl] benzenemethanol

A solution of the product of step (iii) (340mg) in THF (12ml) was added to a solution of 1M borane in THF (3ml) in THF (5-ml) at 0-5° with stirring under nitrogen. The solution was stirred at 5° for 1h. and then at 21° for 1h. Methanol (2ml) was cautiously added and the solution evaporated in vacuo followed by evaporation with methanol (x2). The residue was taken up in methanol (20ml) and sodium hydroxide (500mg) added. The solution was heated at reflux for 0.5h, allowed to stand for 17h at 22°, evaporated in vacuo and the residue partitioned between dichloromethane (60ml) and water (20ml). The organic phase was dried and evaporated in vacuo. The residue was purified by flash chromatography eluting with ethyl acetate to afford the title compound as a colourless viscous gum (85mg).
H.p.l.c. ration of isomers (R:S) 92.7:7.3

(v) (R)-(-)-4-Amino-3,5-dichloro-α-[[[6-[2-(2-pyridinyl)ethoxy] hexyl]amino]methyl]benzenemethanol salt, (E)-butenedioate salt (2:1)

A solution of the product of step (iv) (1.09g) in ethanol (40ml) was treated with ethanolic HCL (3.5ml of 1.15M solution) and hydrogenated over pre-hydrogenated 5% palladium on carbon (0.4g of 50% wet paste) for 24h at room temperature. The mixture was filtered through hyflo, washed with ethanol (25ml), then evaporated to a residue. The residue was partitioned between 8% sodium bicarbonate solution (25ml) and ethyl acetate (2x25ml). The combined extracts were washed with brine (20ml), dried, filtered and evaporated to a crude product. This was taken up in ethyl acetate (25ml) and further washed with 8% sodium bicarbonate solution (25ml) and water (25ml), then dried, decolourised with charcoal, filtered and evaporated to give the crude free base which was recrystallised from isopropyl acetate (5ml) to give purified base (312mg). The base (307mg) in hot isopropanol (1ml) was treated with a hot solution of fumaric acid (42mg) in isopropanol (2ml). This was seeded, cooled, filtered and dried in vacuo to give the title compound (241mg) m.p 126.5-127.5°.
T.l.c toluene:ethanol:ammonia (39:10:1) Rf =0.50
Enantiomeric excess by H.p.l.c = 100%

Example 4

(R)-(-)-4-Amino-3,5-dichloro-α-[[[6-[2-(2-pyridinyl)ethoxy]hexyl] amino]methyl]benzenemethanol, (E)-butenedioate salt (2:1)

(i) (R)-4-Amino-α-(bromomethyl)-3,5-dichlorobenzenemethanol

Borane-THF (1M solution in THF, 10ml) was added slowly to a solution of R-(+)-2-methyl-CBS-oxazaborolidine (288mg) in THF (10-ml) at 5-10° under nitrogen. After 5min. a solution of 1-(4-amino-3,5-dichlorophenyl)-2-bromoethanone (2.4g) in THF (12ml) was added over 5 min. The solution was stined at 5-10° for 10min. then methanol (10ml) slowly added. After 0.5h, the solution was evaporated in vacuo and the residue purified by flash chromatography eluting with dichloromethane to afford the title compound as a crystalline solid (2.37g) m.p. 57-60°
$[\alpha]^{20}_D$= - 35.0° (c 1.0,$CHCl_3$)
H.p.l.c. ratio of enantiomers (R:S) 98.0:2.0

(ii) 6-[2-(2-Pyridinyl)ethoxy]hexanenitrile

A mixture of 2-pyridineethanol (58.5g), 6-bromocapronitrile (100g) aqueous sodium hydroxide (70%w/v, 760ml) and triethylbenzylammonium chloride (TEBAC) (6.49g) was stirred at room temperature under nitrogen for of 90h. Iced water (1 litre) was added to the reaction mixture and extracted with dichloromethane (3x500ml). The combined organic extracts were extracted with 2N hydrochloric acid (3x500ml) and the combined acid washed with dichloromethane (500ml). The acid was adjusted to pH12 with 5N sodium hydroxide (600ml) and extracted with dichloromethane (3x500ml). The combined organic extracts were washed with water (3x500ml), treated with charcoal, dried filtered through hyflo and concentrated in vacuo to give the title compound as a pale green oil (83.25g).
T.1.c. ether:ethanol (10:1) Rf 0.65

(iii) 6-[2-(2-Pyridinyl)ethoxy]hexanamine

A solution of the product of step (ii) (90g) in dry THF (450ml) was added to a stirred solution of borane in THF (1600ml of 1.0M solution) at 0-5°C, over 15 minutes, under nitrogen. The resulting solution was heated to a gentle reflux for 2h. The mixture was cooled to 10-15°C and cautiously treated with ethanol (90ml) added dropwise over 30 minutes. 5.8N HCl (600ml) was then added, again cautiously at first. The resulting mixture was heated to a gentle reflux for 1.5h, then cooled to 40°C. The THF was distilled off under reduced pressure and the aqueous residues made basic with 60%w/v aqueous potassium carbonate solution (750ml). This solution was then extracted with dichloromethane (2x450ml, 1x225ml). The combined extracts were extracted with 2N HCL (600ml, then 300ml) and the combined acid extracts were washed with (180ml) and made basic with 60%w/v aqueous potassium carbonate solution (600ml). The basic aqueous solution was extracted into dichloromethane (2x450ml, 1x225ml) and the combined extracts were dried and concentrated in vacuo to give a colourless oil. The oil (87g) was distilled in batches on a Kugelrohr apparatus, at 175-190°C and 0.2-0.3 to rr, to give the title compound as a colourless oil (69.3g).

T.1.c. dichloromethane:methanol:ammonia (50:8:1) Rf = 0.31

(iv) (R)-4-[2-Bromo-1-[[dimethyl(1,1-dimethylethyl)silyl]oxy]ethyl] 2,6-dichlorobenzenamine

A solution of t-butyldimethylsilyl chloride (TBDMS Cl (2.26g) in dry DMF (10ml) was added to a stirred solution of the product of step (i) (2.85g) and imidazole (0.75g) in dry DMF (4.25ml) under nitrogen, with water bath cooling. The mixture was stirred at room temperature for 19h and water (114ml) and hexane (114ml) were added. The phases were separated and the aqueous phase was re-extracted with hexane (2x28ml). The combined extracts were washed with water (2x28ml) and evaporated in vacuo at 40° to give the title compound (3.9g) as a pale yellow oil.
T.l.c. dichloromethane Rf 0.7.

(v) (R)-4-Amino-3,5-dichloro-α-[[[6-[2-(2-pyridinyl)ethoxy] hexyl]amino methyl]benzenemethanol, (E)-butenedioate salt (2:1)

The product of step (iv) (3.9g) in acetonitrile (25ml) was added to a stirred mixture of the product of step (iii) (3.33g), N,N-diisopropylethylamine (3.5ml) and sodium iodide (1.5g) in acetonitrile (25ml) under nitrogen. The mixture was heated at reflux for 48h, cooled and diluted with ethyl acetate (60ml) and water (30ml). The phases were separated and the aqueous phase was re-extracted with ethyl acetate (2x25ml). The combined extracts were washed with (2x25ml) and evaporated in vacuo at ca 40° to give an orange gum which was dissolved in THF (20ml) and 1M tetrabutylammonium fluoride in THF (14ml) was added. The mixture was stirred at room temperature for 25h and the THF was evaporated off. The residue was partitioned between ethyl acetate (50ml) and water (25ml) and the aqueous phase was re-extracted with ethyl acetate (2x25ml). The combined extracts were washed with water (25ml) and evaporated in vacuo to give an orange gum which was dissolved in isopropanol (5ml) and treated with a hot (60°) solution of fumaric acid (0.58g) in isopropanol (17ml). The solution was stirred and allowed to cool to 30°-40° and sealed. After stirring at 20° for 1h the mixture was cooled to 5° and stirred for 2h. The mixture was stored at 0-5° for 17h and then stirred at room temperature for 2h and filtered. The filter cake was washed with isopropanol (2x5ml) and dried in vacuo at 45° to give the title compound (1.43g) as an off-white solid.
T.1.c. dichloromethane:methanol:ammonia (100:8:1) Rf = 0.25

| | |
|---|---|
| Analysis Found: | C,56.9; H,6.5; N,8.4 |
| $C_{42}H_{58}Cl_4N_6O_4 \cdot C_4H_4O_4$ requires: | C,57.0; N,6.45; N,8.7% |

(vi) (R)-4-Amino-3,5-dichloro-α-[[[6-[2-(2-pyridinyl) ethoxy]hexyl]amino] ethyl]benzenemethanol,(E)- butenedioate salt (2:1),alternative synthesis

The product of step (i) (10.07g) and imidazole (7.22g) in dry DMF (150ml) were treated with chlorotriethylsilane (6.3ml) and allowed to stand for 1h. Further chlorotriethylsilane (2.7ml) was added and after 0.5h the solution was evaporated in vacuo to 50ml. The residue was partitioned between 8% sodium hydrogen carbonate (150ml) and ether (300ml) and the ether extract rapidly washed with brine (100ml), dried and evaporated in vacuo. The resulting oil in acetonitrile (50ml) was added to a mixture of the product of step (iii) (9.90g), sodium hydrogen carbonate (5.93g), sodium iodide (5.3g) and acetonitrile (200ml) and stirred at reflux under nitrogen for 65h. The mixture was cooled, filtered and the filtrate evaporated in vacuo. The residue in methanol (40ml) was treated with potassium carbonate (10g), stirred at 21° for 6h, filtered through hyflo and evaporated in vacuo. The residue in ethyl acetate (300ml) was washed with water (50ml) and brine (50ml), dried and evaporated in vacuo. The resultant dark oil was purified by flash chromatography eluting with toluene:triethylamine (98:2) and a gradient of toluene:ethanol:triethylamine (97:1:2-96:2:2- 93:5:2) giving firstly impurities then slightly impure product. This material was taken up in methanol (40ml) and fumaric acid (746mg) added. Evaporation in vacuo and recrystallisation of the residue from isopropanol (40 ml) afforded the title compound as a colourless powder (2.43g) m.p.127-129°

Analysis Found C,56.55; H,6.4;N,8.4;Cl,14.6.

$C_{21}H_{29}Cl_2N_3O_2.0.5C_4H_4O_4$ requires: C,57.0;H,6.45;N,8.7;Cl,14.6%

$[\alpha]_D^{20}$ = -25.5°(c. 1.0,methanol)

H.p.l.c. ratio of enantiomers (R:S) 99.1 : 0.9

Example 5

(R)-(-)-4-Amino-3,5-dichloro-α-[[[6-[2-(2-pyridinyl)ethoxy]hexyl] amino]methyl] benzenemethanol,(E)-butenedioate salt (2:1)

(i) 1-[4-Amino-3,5-dichlorophenyl]-2-azidoethanone

A mixture of 1-[4-amino-3,5-dichlorophenyl]-2-bromoethanone (28.3g) and sodium azide (7.0g) in ethanol (150ml), water (50ml), and THF (200ml) was stirred at room temperature for 1 hour. The solution was poured into brine (500ml) and ethyl acetate (500ml). The aqueous phase was extracted with ethyl acetate (200ml). The combined organic extracts were washed with water (500ml), dried and evaporated under reduced pressure. The residue was triturated with propan-2-ol (150ml) and dried to yield the title compound as a yellow powder (23.0g) m.p. 115-6°C

(ii) (R)-4-Amino-α-(azidomethyl)-3,5-dichlorobenzenemethanol

A solution of the product of step (i) (2.45g) in dry THF (10 ml) was added over 2 minutes to a solution of R-(+)-2-methyl-CBS-oxazaborolidine (0.288g) in dry THF (10ml) containing a 1M solution of borane in THF (6 ml) at 10°C. The solution was stirred at room temperature for 10 minutes and a further portion of borane (4ml) was added. After stirring for a further 5 minutes, methanol (5ml) was cautiously added and the solution was evaporated under reduced pressure. The residue was chromatographed eluting with dichloromethane and appropriate eluates were collected and evaporated under reduced pressure to give a white solid (2.43g).
$[\alpha]_D^{20}$ = -78.7° (c.0.011,methanol)
H.p.l.c. ratio of enantiomers (R:S) 91:9
The sample was recrystallised from propan-2-ol(15ml) and water (15ml) to give a white flaky solid (0.6g) with a reduced enantiomeric excess of the (R)-enantiomer.
$[\alpha]_D^{20}$ = -26.9° (c.0.007,methanol)
H.p.l.c. ratio of enantiomers (R:S) 61:39
However, concentration of the mother liquors to about 20ml gave the title compound as pale yellow agglomerates (1.7g)
T.1.c. dichloromethane Rf: 0.13
$[\alpha]_D^{20}$ = -100.9° (c.0.01,methanol)
H.p.l.c. ratio of enantiomers (R:S) 100:0

(iii)(R)-4-Amino-α-(aminomethyl)-3,5-dichlorobenzenemethanol

A mixture of the product of step (ii) (2.47g) and 5% palladium on carbon (0.25g) in ethanol (50ml) was stirred under an atmosphere of hydrogen for 1.5 hours. The suspension was filtered through a pad of hyflo and the filtrate was evaporated to yield the title compound as a white solid (2.2g).
T.l.c. dichloromethane:ethanol:ammonia (30:8:1) Rf 0.39

(iv) (R)-N-[1-(4-Amino-3,5-dichlorophenyl)-1-hydroxyethyl]-6-[2-(2-pyridinyl)ethoxy]hexanamide

N,N'-Carbonyldiimidazole (0.78g) was added to a solution of 6-[2-(2-pyridinyl)ethoxy]hexanoic acid (24g) in dry THF (12ml). After stirring at room temperature for 30 minutes, a solution of the product of step (iii) (1.05g) in dry THF (10ml) was added. After stirring at room temperature for 1 hour, the solution was evaporated under reduced pressure and the residue was partitioned between ethyl acetate (100ml) and water (100mol). The phases were separated and the organic phase was washed with sodium carbonate (100ml) and water (100ml) and dried. Evaporation of the solvent gave the title compound as a clear oil (1.3g)
T.1.c. toluene:ethanol:triethylamine (36:2:1), Rf 0.26

(v) (R)-4-Amino-3,5-dichloro-α-[[[6-[2-(2-pyridinyl)ethoxy]hexyl] amino]methyl]benzenemethanol, (E)-butenedioate (salt) (2:1)

A 1.0M solution of borane in THF (8.8ml) was added to a solution of the product of step (iv) (1.28g) in dry THF (25ml) and was heated under reflux for 18 hours. A solution of potassium hydroxide (1.48g) in methanol (50ml) was added and the mixture was heated under reflux for 2 hours. The solution was evaporated under reduced pressure and the residue was partitioned between ethyl acetate (50ml) and pH6.5 buffer (100ml). The aqueous phase was extracted with ethyl acetate (50ml). The combined organic extracts were dried and evaporated under reduced pressure. The residue was chromatographed eluting with dichloromethane:ethanol:ammonia (150:8:1) and appropriate eluates were collected and evaporated to give the free base of the title compound as an oil (0.68g).
H.p.l.c. ratio of enantiomers (R:S) 97:3
The oil was dissolved in propan-2-ol (7ml) and a solution of fumaric acid (0.092g) in propan-2-ol (2ml) was added. The material failed to crystallise. The solution was evaporated and the residue was triturated under ethyl acetate (15ml) to yield the title compound as a white powder (0.75g).
T.1.c. dichloromethane:ethanol:ammonia (60:8:1) Rf = 0.51
Enantiomeric excess by H.p.l.c. >90% (R)-enantiomer.

Example 6

Preparation of (R)-4-amino-α-(aminomethyl)-3,5-dichlorobenzenemethanol, a chiral precursor to the (R)-isomer of Compound A

(i) (R)-4-Amino-α-(aminomethyl)-3,5-dichlorobenzenemethanol, (2S,3S)-di-p-toluoyl-D-tartaric acid salt

A solution of 4-amino-α-(aminomethyl)-3,5-dichlorobenzenemethanol (4.42g) in hot ethanol (80ml) was added to a solution of di-p-toluoyl-D-tartaric acid (4.04g) in hot ethanol (80ml). On prolonged standing white needles deposited and were collected and dried (2.8g). m.p. 168-169°C.
$[\alpha]_D^{20}$ = +47.15° (methanol)
The sample (2.7g) was recrystallised from ethanol (20ml) yielding the title compound as white microneedles (1.6g). m.p. 167-168°C.
$[\alpha]_D^{20}$ = +38.45° (methanol)
The remainder of the sample (1.58g) was recrystallised from ethanol (120ml) yielding white needles (1.1g). m.p. 167-168°C.
$[\alpha]_D^{20}$ = +39.8° (methanol).
H.p.l.c. ratio of enantiomers (R:S) 95.1:4.9

(ii) (R)-4-Amino-α-(aminomethyl)-3,5-dichlorobenzenemethanol

The product of step (i) (2.1g) was partitioned between IN sodium carbonate (100ml) and ethyl acetate (100ml). The phases were separated and the aqueous phase was extracted with ethyl acetate (3x50ml). The combined organic extracts were dried and evaporated to give the title compound as a clear oil (1.1g)
T.1.c. dichloromethane:ethanol:ammonia (30:8:1) Rf = 0.39
$[\alpha]_D^{22}$ = -32.7° (c. 0.01, methanol)

## Claims

1. The compound (R)-(-)-4-amino-3,5-dichloro-α-[[[6-[2-(2-pvridinyl)ethoxy]hexyl]amino]methyl]benzenemethanol, or a physiologically acceptable salt thereof.

2. The compound according to claim 1 or a physiologically acceptable salt thereof for the manufacture of a medicament for the treatment, relief or prevention of a condition which may be ameliorated by a compound having selective $\beta_2$-adrenoreceptor stimulant activity.

3. A pharmaceutical formulation comprising the compound according to claim 1 or a physiologically acceptable salt thereof together with a physiologically acceptable carrier or excipient.

4. A method for the preparation of the compound according to claim 1 or a physiologically acceptable salt thereof, which comprises

(A) deprotecting a compound of formula (I)

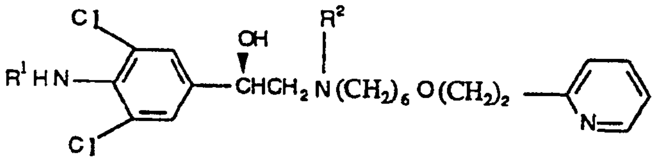

(I)

wherein $R^1$ and $R^2$ each independently represents a hydrogen atom or a protecting group with the proviso that at least one of them represents a protecting group; or

(B) reacting a compound of formula (XI)

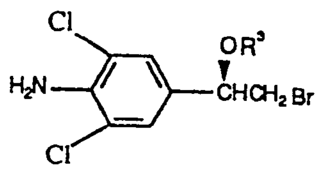

(XI)

(wherein $R^3$ is a hydroxyl protecting group) with a compound of formula (XII)

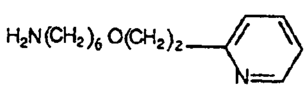

(XII)

followed by removal of any protecting groups where present; or

(C) reducing a compound of formula (XIV)

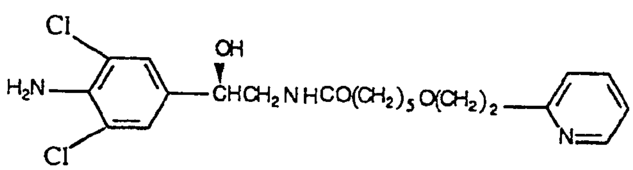

(XIV)

or a protected derivative thereof, followed by removal of any protecting groups where present; or

(D) alkylating a compound of formula (XVIII)

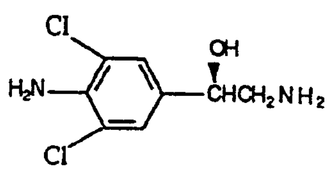

(XVIII)

with an alkylating agent of formula (XIX)

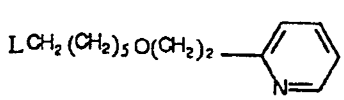

(XIX)

(wherein L represents a leaving group),

or with a compound of formula (XX)

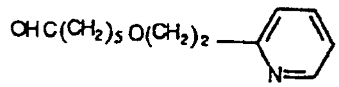

(XX)

in the presence of a reducing agent, followed by removal of any protecting groups where present; followed in each case, if desired, by the conversion of the resulting compound or a salt thereof into a physiologically acceptable salt thereof.

5. A method according to claim 4 wherein $R^2$ is an (R)-2-hydroxy-1-phenylmethyl group.

6. A method according to claim 4 wherein $R^3$ is a silyl ether group such as a triethylsilyl group or a t-butyldimethylsilyl group.

European Patent Office

# EUROPEAN SEARCH REPORT

Application Number EP 91305057.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | GB - A - 2 187 734 (GLAXO) * Claims 1,9,11,12; example 3 * | 1-4 | C 07 D 213/30<br>A 61 K 31/44 |
| A | GB - A - 2 165 542 (GLAXO) * Claims 1,10,11 * | 1-4 | |

TECHNICAL FIELDS SEARCHED (Int. Cl.5)

C 07 D 213/00

The present search report has been drawn up for all claims

| Place of search | Date of completion of the search | Examiner |
|---|---|---|
| VIENNA | 14-08-1991 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)